(19) **Europäisches Patentamt**

**European Patent Office**

**Office européen des brevets**

(11) Veröffentlichungsnummer: **0 465 653 A1**

(12) **EUROPÄISCHE PATENTANMELDUNG**
**veröffentlicht nach Art. 158 Abs. 3 EPÜ**

(21) Anmeldenummer: **90906432.1**

(22) Anmeldetag: **03.04.90**

(86) Internationale Anmeldenummer:
**PCT/SU90/00082**

(87) Internationale Veröffentlichungsnummer:
**WO 90/11992 (18.10.90 90/24)**

(51) Int. Cl.⁵: **C07C 53/126**, C07C 51/41,
//C08L21/00,C08L7/00,
C08K5/09

(30) Priorität: **04.04.89 SU 4674492**

(43) Veröffentlichungstag der Anmeldung:
**15.01.92 Patentblatt 92/03**

(84) Benannte Vertragsstaaten:
**DE FR IT NL**

(71) Anmelder: **VSESOJUZNY NAUCHNO-ISSLEDOVALESKY I KONSTRUKTORSKO-TEKHNOLOGICHESKY INSTITUT OBORUDOVANIA NEFTEPERABATYVAJUSCHEI I NEFTEKHIMICHESKOI PROMYSHLENNOSTI Volgograd, 400085(SU)**

Anmelder: **VOLZHKOE PROIZVODSTVENNOE OBIEDINENIE "ORGSINTEZ"IMENI 60 LETIA SSSR Volgogradskaya obl., Volzhsky, 404117(SU)**

(72) Erfinder: **KOSTJUCHENKO, Vladimir Mitrofanovich ul. Komsomolskaya, 10-188 Volgograd, 400066(SU)**
Erfinder: **NO, Elena Borisovna**

**pr. Lenina, 20-84 Volgograd, 400066(SU)**
Erfinder: **SHVARTS, Arkady Grigorievich ul. Abelmanovskaya, 7-20 Moscow, 109147(SU)**
Erfinder: **FROLIKOVA, Valentina Georgievna shosse Entuziastov, 13-32 Moscow, 111024(SU)**
Erfinder: **KOZHEVNIKOV, Vasily Sergeevich ul. Engelsa, 19-46 Volgogradskaya obl. Volzhsky, 404111(SU)**
Erfinder: **KULESHENKO, Mikhail Ivanovich ul. Lenina, 97-423 Volgogradskaya obl. Volzhsky, 404120(SU)**
Erfinder: **RYKOV, Vyacheslav Karpovich ul. Kommunisticheskaya, 30-15 Volgogradskaya obl. Volzhsky, 404130(SU)**

(74) Vertreter: **von Füner, Alexander, Dr. Patentanwälte v. Füner, Ebbinghaus, Finck Mariahilfplatz 2 & 3 W-8000 München 90(DE)**

(54) **VERFAHREN ZUR HERSTELLUNG EINES DIE ADHÄSION ZWISCHEN KAUTSCHUK UND METALLDRAHT VERBESSERNDEN MITTELS.**

(57) Verfahren zur Herstellung eines Promotors der Adhäsion für Befestigung von Gummi an eine Drahtlitze besteht darin, daß man das Verschmelzen organischer Säuren gewählt aus, der Fettsäure mit 5 bis 25 Kohlenstoffatomen, oder ihrer Gemische, oder Tall- oder Naphthensäuren mit basischem Kobaltkarbonat in Gegenwart der Essigsäure und des Alkylphenolaminharzes, das eine Erweichungstemperatur von 60 bis 130°C und einen Gehalt an Stickstoff von 0,5 bis 3,5 Masse% aufweist, mit anschließender Entfernung der Essigsäure

vornimmt.

Gebiet der Technik

Die vorliegende Erfindung bezieht sich auf organische Chemie und betrifft insbesondere einen Adhäsionspromotor für Befestigung von Gummi an eine Drahtlitze.

Zugrundeliegender Stand der Technik

Bekannt ist eine Verfahren zur Herstellung eines Adhäsionspromotors von Gummi an eine Drahtlitze aus Kobaltseifen (Kobaltnaphthenat, Kobaltstearat), des von der japanischen Firma Nippon Mining Company Limited (Annual Report, 1985) entwickelt wurde. Das Verfahren besteht darin, daß man die Verseifung organischer Säuren (Naphthen- und Stearinsäuren) mit einem Alkali unter Entstehung von Natriumseife vornimmt. Dann wird die Austauschreaktion zwischen der Natriumseife einer organischen Säure und einem anorganischen Kobaltsalz, das in der wässerigen vorhanden ist, in Gegenwart eines organischen Lösungsmittels durchgeführt.

Das Reaktionsprodukt ist Kobaltseife, die sich in organischer Phase befindet, und ein anorganisches Natriumsalz, das sich in wässeriger Phase befindet. In der wässerigen Phase sind außerden vorhanden: das nichtumgesetzte anorganische Kobaltsalz (ein toxischer Stoff), teilweise aufgelöste organische Säuren und ihre Natriumseifen. Die wässerige Phase wird abgetrennt und man nimmt das Waschen der organischen Phase mit frischem Wasser mit dem Ziel der Entfernung der Rückstände des anorganischen Natriumsalzes aus ferselben vor, das Vorhandensein des letzteren im Endprodukt verschlechtert seine Qualität.

Infolge des Waschvorganges fällt Spülwasser an, das die obengenannten Stoffe aufweist.

Aus der gewaschenen organischen Phase wird das organische Lösungsmittel zusammen mit Wasserrückständen abgetrieben. Im Rückstand erhält man ein Endprodukt, das einen festen Stoff mit dunkelviolettem Farbton und folgenden Eigenschaften darstellt: die Erwichungstemperatur beträgt, beispielsweise, für Kobaltnaphthenat 79°C, für Kobaltstearat 88°C der Massenanteil an Kobalt für Kobaltnaphthenat beträgt 10,2%, für Kobaltstearat - 9,4%; der Masseanteil an den im Toluol unlöslischen Stoffe beträgt für das Kobaltnaphthenat und -stearat unter 1,0%; der Masseanteil an flüchtigen Stoffen beträgt für Kobaltnaphthenat 0,3% und für Kobaltstearat unter 0,4%.

Das genannte Verfahren zeichnet, sich durch Mehrstufigkeit der Prozeßführung, durch das Vorliegen einer großen Menge von Abwasser, die Beimengungen organischer Stoffe, anorganischer Natrium- und Kobaltsalze, darunter toxischer Beimengungen, enthalten, sowie durch die Möglichkeit der Verunreinigung des Endproduktes mit Natriumsalzen aus, was negativ auf die Qualität von Reifen und technischen Gummierzeugnissen auswirkt.

Bekannt ist ebenfalls ein Verfahren zur Herstellung von Tallaten (SU, A, 755825), das darin besteht, daß man ein Gemisch aus organischen Säuren des Kolophoniums oder der Tallöls bei einer Temperatur von 200 bis 275°C mit Oxyden oder Hydroxyden, oder Azetaten oder Karbonaten der Metalle, gewählt aus der Gruppe: Zink, Kalzium, Blei, Mangan, Kobalt, Eisen, Aluminium, oder ihre Gemische in Gegenwart eines Zusatzstoffes unsetzt, als solcher verwendet man Amin, gewählt aus der Gruppe: Hexamethylentetramin oder Melamin, oder Polyäthylenpolyamin, in einer Menge von 0,2 bis 5%, bezogen auf das Gewicht des Kolophoniums oder des Tallöls. Die im genannten Verfahren herzustellenden Produkte weisen einen niedrigen Gehalt an Kobalt (4,5 Masse%) und eine niedrige Erweichungstemperatur (von 40 bis 80°C) auf, weshalb sie keine Anwendung als Promotore der Adhäsion für die Befestigung von Gummi an eine Drahtlitze finden.

Offenbarung der Erfindung

Der Erfindung liegt die Aufgabe zugrunde, durch die Änderung der Bedingungen der Prozeßführung die Technologie zu vereinfachen, indem man die Mehrstufigkeit ausschließt, die Entstehung von Abwasser zu reduzieren und die Qualität des Endproduktes zu verbessern.

Die Aufgabe wird dadurch gelöst, daß man im Verfahren zur Herstellung eines Adhäsionspromotors fur die Befestigung von Gummi an eine Drahtlitze durch Umsetzung organischer Säuren mit basischem Kobaltkarbonat in Gegenwart von Amin unter Anfallen des Endproduktes, erfindungsgemäß als organische Säuren eine Fettsäure mit 5 bis 25 Kohlenstoffatomen oder ihre Gemische, Tallsäuren oder Naphthensäuren verwendet und als Amin das Alkylphenolaminharz verwendet, das eine Erweichungstemperatur von 60 bis 130°C und einen Stickstoffgehalt von 0,5 bis 3,5 Masse% hat, und die Umsetzung der organischen Säuren mit dem basischen Kobaltkarbonat durch ihr Verschmelzen in Gegenwart von Essigsäure in Kombination mit dem Alkylphenolaminharz mit anschließender Entfernung der Essigsäure vornimmt.

Zweckmäßigerweise sollen die organischen Säuren in einer Menge von 1 bis 3 Mol je 1 g-atom des

3

Kobalts genommen werden, das im basischen Kobaltkarbonat enthalten ist. Die Prozeßführung in Gegenwart der Essigsäure bewirkt eine vollständige Umwandlung des abasischen Kobaltkarbonats und die Einführung von Kobalt in das Endprodukt in einer Menge, die dem theoretischen Wert naheliegt. Vorzugsweise soll Eisessig oder eine 70 bis 99%ige wässerige Lösung der Essigsäure verwendet werden. Das Verschmelzen soll vorzugsweise bei einer Temperatur von 140 bis 190°C vorgenommen werden.

Das erfindungsgemäße Verfahren ermöglicht es:

- die Technologie der Prozeßführung durch Ausschließung von der Mehrstufigkeit zu vereinfachen;
- die Menge von Abwasser bis auf ein Minimum zu bringen (etwa 0,1 Masseanteil je 1 Masseanteil des Endproduktes);
- die anfallenden Abwässer (infolge des Fehlens anorganischer Produkte, darunter von toxischen Stoffen) durch Rektifikation oder Verbrennung leicht zu verwerten oder unschädlich zu machen;
- ein Endprodukt herzustellen, das eine höhere Erweichungstemperatur aufweist, wodurch es sich leichter granulieren und zerkleinern läßt, es klumt sich weniger zusammen und läßt sich beim Einsatz in der Herstellung von technischen Gummierzeugnissen leicht verarbeiten;
- den Gehalt des Fertigproduktes an Kobalt (von 10 bis 15 Masse%) im Vergleich zum Promotor der japanischen Firma Nippon Mining Company (von 8,5 bis 10,5 Masse%) zu steigern, was eine sparsamere Verwendung von Promotoren bewirkt, da bei der Zusammenstellung von Rezepturen von Gummigemischen die Menge des Promotors der Adhäsion unter Berücksichtigung des Gehalts an Kobalt in demselben berechnet wird.

Beste Ausführungsvariante der Erfindung

Das erfindungsgemäße Verfahren wird wie folgt ausgeführt.

In einen Reaktor gibt man berechnete Mengen organischer Säuren und des besischen Kobaltkarbonats auf. Zweckmäßigerweise sollen die organischen Säuren in einer Menge von 1 bis 3 Mol je 1 g-atom des Kobalts genommen werden, das im basischen Kobalt enthalten ist. Als organische Säuren verwendet man eine Fettsäure mit 5 bis 25 Kohlenstoffatomen oder ihre Gemische, oder Tallsäuren, oder Naphthesäuren. Das Gemisch läßt man unter Vermischen verschmelzen. Die Temperatur des Verschmelzens hängt von der Natur der organischen Ausganssäuren ab. Vorzugsweise soll die Prozeßführung bei einer Temperatur von 140 bis 190°C erfolgen. Die Prozeßführung erfolgt in Gegenwart der Essigsäure und des Alkylphenolaminharzes. Vorzugsweise verwendet man Eisessig oder eine 70 bis 99%ige wässerige Essigsäurelösung.

Die Essigsäure sichert eine vollständige Umwandlung des basischen Kobaltkarbonats und bewirkt die Einführung von Kobalt in das Endprodukt in einer Menge, die dem theoretischen Wert naheliegt.

Die Einführung des Alkylphenolaminharzes ermöglicht es, die Erweichungstemperatur des Endproduktes und den Gehalt an Kobalt in demselben in gewünschter Richtung zu korrigieren. Man verwendet das Alkylphenolaminharz, das eine Erweichungstemperatur von 60 bis 130°C und einen Gehalt an Stickstoff von 0,5 bis 3,5 Masse% hat. Das bei der Prozeßführung anfallende Wasser wird in einem der zwei nachstehend angeführten Verfahren abgetrieben:

- direktes Abtrieben eines Gemisches aus Wasser und Essigsäure mit kontinuierlicher Kompensation des Verlusttes an Essigsäure aus der Reaktionsmasse durch die Zuführung neuer Portionen von Essigsäure;
- unter Zuhilfenahme einer Rektifikationskolonne mit einer theoretischen Bodenzahl von 15 bis 20, die über einem Reaktor aufgestellt wird. Dabei wird vom oberen Teil der Kolonne das Wasser abgetrieben, und die Essigsäure, die aus der Reaktionsmasse verdampft, wird aus Kolonnenblase dem Reaktor zurückgeführt.

Nach der Beendigung der Auscheidung von Reaktionswasser, werden die Essigsäure und der Überschuß an organischen Säuren (falls dieser zu verzeichnen ist) zunächst bei atmosphärischem Druck und dann in Vakuum bei einer Temperatur der Reaktionsmasse von 150 bis 190°C und einem Restdruck von 0,05 bis 0,5 kp/cm$^2$ (0,005 bis 0,05 MPa) innerhalb von 1 bis 5 Stunden abgetrieben.

Die Schmelze des Endproduktes wird abgegossen beziehungsweise bei der Ausgangstemperatur der Schmelze von 150 bis 200°C granuliert.

Das Endprodukt stellt einen festen Stoff vom dunkelroten oder dunkelviolettem bis zum schwarzen Farbton dar, das wasserunlöslich ist, löslich in organischen Lösungsmitteln (Benzin, White Sprit, Toluol, Benzol, Chloroform und anderen) ist, das Produkt weist eine Erweichungstemperatur von 90 bis 140°C und einen Gehalt an Kobalt von 10 bis 15 Masse% auf.

Zum besseren Verstehen der vorliegenden Erfindung werden nachstehende Beispiele für die Ausführung des erfindungsgemäßen Verfahrens angeführt.

Beispiel 1

In einen Reaktor gibt man 100 g (0,829 Mol) Gemisch aus synthetischen Fettsäuren mit 5 bis 8 Kohlenstoffatomen mit einer Säuerzahl von 465 mk KOH/g und 32,5 g (0,276 g-atom Co) des besischen Kobaltkarbonats mit einem Gehalt an Kobalt von 50,1 Masse% auf.

Das Gemisch wird unter Vermischen auf 150 bis 160°C erhitzt und bei dieser Temperatur unter Abdestillation des Reaktionswassers stehengelassen. Nach der Beendigung der Abdestillation des Wassers setzt man der Reaktionsmasse 25 g Alkylphenolaminharz, das eine Erweichungstemperatur von 80°C und einen Massenanteil von Stickstoff 2,5% hat, und eine solche Menge des Eisessigs zu, die das Sieden der Reaktionsmasse bei einer Temperatur von 140 bis 150°C sicherstellt.

Die Reaktionsmasse wird bei dieser Temperatur vermischt, indem man das erneut anfallende Reaktionswasser zusammen mit der Essigsäure abdestilliert, die Verluste an Essigsäure aus der Reaktionsmasse werden durch den Zusatz neuer Portionen der Essigsäure kompensiert.

Die Behandlung der Reaktionsmasse mit Essigsäure wird bis zur Beendigung der Ausscheidung von Reaktionswasser fortgeführt, wovon man nach der Konzentration der zuzuführenden und abzudestillierenden Essigsäure urteilt. Die Gesamtmenge der verwendeten Essigsäure beträgt 83 g.

Danach wird die Essigsäure und der Überschuß an Fettsäuren aus der Reaktionsmasse abdestilliert, zunächst bei atmosphärischem Druck und dann in Vakuum abgetrieben, die Schmelze wird bei einer Temperatur von 150 bis 160°C und einem Restdruck von 0,2 kp/cm$^2$ (0,02 MPa) innerhalb 1 Stunde stehengelassen.

Die Schmelze des Endproduktes wird aus dem Reaktor abgegossen beziehungsweise granuliert. Die Ausbeute Endprodukt beträgt 102,1 Masse%. Physikalische Kenndaten des hergestellten Produktes sind in der nachstehenden Tabelle angeführt.

Beispiel 2

Die Prozeßführung erfolgt analog der in Beispiel 1 beschriebenen, wobei man 100 g (0,695 Mol) Gemisches aus synthetischen Fettsäuren mit 6 bis 10 Kohlenstoffatomen von mit einer Säurezahl von 390 mg KOH/g, 34,1 g (0,290 g-atom Co) des basischen Kobaltkarbonats mit einem Gehalt an Kobalt von 50,1 Masse%, 30 g Alkylphenolaminharzes mit einer Erweichungstemperatur von 90°C und einem Masseanteil an Stickstoff von 2,3%, 100 g einer 95%igen wässrigen Lösung der Essigsäure verwendet. Die Temperaturführung der Reaktion liegt im Bereich von 150 bis 170°C. Die Ausbeute am Fertigprodukt beträgt 126 g (97 Masse%). Physikalische Kenndaten des Fertigproduktes sind in der nachstehenden Tabelle aufgeführt.

Beispiel 3

Die Prozeßführung erfolgt analog der in Beispiel 1 beschriebenen, wobei man 100 g (0,446 Mol) Gemisch aus synthetischen Fettsäuren mit 9 bis 17 Kohlenstoffatomen in einem Molekül, mit einer Säurezahl von 250 mg KOH/g, 25,75 g (0,223 g-atom Co) basisches Kobaltkarbonat mit einem Gehalt an Kobalt von 51,0 Masse%, 10 g Alkylphenolaminharz mit einer Erweichungstemperatur von 100°C und einem Masseanteil an Stickstoff von 2,2%, 100 g 80%ige wässeriger Lösung der Essigsäure verwendet.

Die Abtreibung von Wasser erfolgt bei einer Temperatur von 180°C, die Behandlung der Essigsäure erfolgt bei einer Temperatur von 160 bis 180°C. Das Vakuumieren führt man bei einer Temperatur von 170 bis 180°C und einem Restdruck von 0,1 bis 0,2 kp/cm$^2$ (0,01 bis 0,02 MPa innerhalb einer Stunde durch.

Man erhält 121,5 g (99 Masse%) Endprodukt. Physikalische Kenndaten des Endproduktes sind an der nachstehenden Tabelle angeführt.

Beispiel 4

Die Prozeßführung erfolgt analog der in Beispiel 3 beschriebenen. Man verwendet 100 g (0,437 Mol) Naphthensäuren mit einer Säuerzahl von 245 mg KOH/g, 26,05 g (0,230 g-atom Co) des basischen Kobaltkarbonates mit einem Gehalt an Kobalt von 52 Masse%, 5 g Alkylphenolaminhärz mit einer Erweichungstemperatur von 92°C und einem Masseanteil an Stickstoff von 2,5 %, 120 g 70%igen wässerige Lösung der Essigsäure. Die Ausbeute an Endprodukt macht 118,0 g (99 Masse%) aus. Physikalische Kenndaten des Endproduktes sind in der nachstehenden Tabelle angeführt.

Beispiel 5

In einen Reaktor gibt man 100 g (0,330 Mol) Tallfettsäuren mit 17 bis 22 Kohlenstoffatomen in einem Molekül und einer Säurezahl von 185 mg KOH/g sowie 24,4 g (0,219 g-atom Co) basisches Kobaltkarbonat mit einem Gehalt an basischem Kobalt karbonat von 53 Masse% auf.

Das Gemisch wird unter Vermischen auf 180°C erhitzt und bei dieser Temperatur unter Abtreibung des Reaktionswassers stehengelassen.

Nach der Beendigung der Abtreibung von Wasser setzt man der Reaktionsmasse eine solche Menge der Essigsäure zu, damit das Sieden bei einer Temperatur der Reaktionsmasse von 170 bis 180°C zustandekommt.

Das Gemisch wird bei dieser Temperatur vermischt, wobei das erneut anfallende Reaktionswasser unter Zuhilfenahme einer Rektifikationskolonne mit theoretischem Bodenzahl von 17 abgetrieben wird, die Kolonne wird über dem Reaktor montiert. Die aus der Reaktionsmasse verdampfende Essigsäure wird dabei aus der Kolonnenblase dem Reaktor zurückgeführt.

Nach der Beendigung der Abtreibung von Wasser setzt man der Reaktionsmasse 5 g Alkylphenolaminharz mit einer Erweichungstemperatur von 130°C und einem Masseanteil an Stickstoff von 0,5% zu, es wird das direkte Abtreiben (nicht durch die Kolonne) der Essigsäure bei einer Temperatur von 170 bis 190°C vorgenommen, indem man den Restdruck bis 0,2 - 0,4 kp/cm$^2$ (0,02 bis 0,04 MPa) schrittweise herabsetzt und das Reaktionsgemisch bei diesem Druck innerhalb von 1 bis 2 Stunden stehenläßt. Man erhält 120,6 g (97 Masse%) des Endproduktes. Physikalische Kenndaten des Endproduktes sind in der nachstehenden Tabelle angeführt.

Beispiel 6

Die Prozeßführung erfolgt analog der in Beispiel 5 beschriebenen, wobei 100 g (0,303 Mol) Gemisch aus synthetischen Fettsäuren mit 19 bis 25 Kohlenstoffatomen in einem Molekül mit einer Säuerzahl von 170 mg KOH/g, 35,7 g (303 g-atom Co) basisches Kobaltkarbonat mit einem Gehalt an Kobalt von 50 Masse%, 35 g Alkylphenolaminharz mit einer Erweichungstemperatur von 60°C und einem Masseanteil an Stickstoff von 3,5% und Eisessig verwendet werden. Die Ausbeute an Endprodukt beträgt 167,0 g (98 Masse%). Physikalische Kenndaten des Endproduktes sind in der nachstehende Tabelle angeführt.

Beispiel 7

Die Prozeßführung erfolgt analog der in Beispiel 5 beschriebenen, wobei 100 g (0,348 Mol) Stearinsäure mit einer Säurezahl von 195 mg KOH/g, 23,9 g (0,205 g-atom Co) basisches Kobaltkarbonat mit einem Gehalt an Kobalt von 50,5 Masse%, 3 g Alkylphenolaminharz mit einer Erweichungstemperatur von 104°C und einen Masseanteil an Stickstoff von 1,8% und 90%ige wässerige Lösung der Essigsäure verwendet werden. Die Ausbeute an Endprodukt beträgt 116,3 g (98 Masse%). Physikalische Kenndaten des Endproduktes sind in der nachstehenden Tabelle angeführt.

## Tabelle

### Physikalische Kenndaten der hergestellten Promotoren im Vergleich zu den Promotoren der japanischen Firma Nippon Mining Company

| lfd. Nr. | Nr. der Beispiele | Erweichungs-temperatur, °C | Masseanteil, % | | | Ausbeute | |
|---|---|---|---|---|---|---|---|
| | | | Kobalt | in Toluol unlösliche Stoffe | flüchtige Stoffe, g | %, bezogen auf den theoretischer Wert | |
| 1 | Beispiel 1 | 133 | 15,15 | 0,02 | 0,64 | 102,1 | 95 |
| 2 | Beispiel 2 | 118 | 13,17 | 0,01 | 0,52 | 126,0 | 97 |
| 3 | Beispiel 3 | 105 | 10,70 | 0,03 | 0,23 | 121,5 | 99 |
| 4 | Beispiel 4 | 110 | 11,25 | 0,04 | 0,28 | 118,0 | 99 |
| 5 | Beispiel 5 | 98 | 10,40 | 0,05 | 0,35 | 120,6 | 97 |
| 6 | Beispiel 6 | 92 | 10,38 | 0,12 | 0,14 | 167,0 | 98 |
| 7 | Beispiel 7 | 103 | 10,17 | 0,06 | 0,21 | 116,3 | 98 |
| 8 | Kobaltnaphthenat (Promotor der japanischen Firma) | 79 | 10,2 | unter 1,0 | 0,3 | – | – |
| 9 | Kobaltstearat (Promotor der japanischen Firma) | 88 | 9,4 | unter 1,0 | 0,4 | – | – |

## Gewerbliche Anwendbarkeit

Die gemäß dem beanspruchten Verfahren herzustellenden Promotoren der Adhäsion für die Befestigung von Gummi an eine Drahtlitze werden als Ingredienzien von Gummigemischen bei der Herstellung von technischen Gummierzeugnissen, die mit Drahtlitze verstärkt sind (Reifen, Gummifördergurte, Schläuche und andere Erzeugnisse), verwendet.

## Patentansprüche

1. Verfahren zur Herstellung eines Promotors der Adhäsion für Befestigung von Gummi an eine Drahtlitze durch Umsetzung organischer Säuren mit basischem Kobaltkarbonat in Gegenwart des Amins unter Anfallen eines Endproduktes, dadurch **gekennzeichnet**, daß man als organische Säuren Fettsäure mit 5 bis 25 Kohlenstoffatomen, oder ihre Gemische, Tall- oder Haphthensäuren verwendet und als Amin das Alkylphenolaminharz einsetzt, das eine Erweichungstemperatur von 60 bis 130°C und einen Gehalt an Stickstoff von 0,5 bis 3,5 Masse% hat, und die Umsetzung der organischen Säuren mit dem basischen Kobaltkarbonat durch ihr Verschmelzen in Gegenwart der Essigsäure in Kombination mit Alkylphenolaminharz mit anschließender Entfernung der Essigsäure vornimmt .

**2.** Verfahren nach Anspruch 1, dadurch **gekennzeichnet**, daß man die organischen Säuren in einer Menge von 1 bis 3 Mol je 1 g-atom Kobalt nimmt.

**3.** Verfahren nach Anspruch 1, dadurch **gekennzeichnet**, daß man Eisessig beziehungsweise eine 70 bis 99%ige wässerige Lösung der Essigsäure verwendet.

**4.** Verfahren nach Anspruch von 1 bis 3, dadurch **gekennzeichnet**, daß man das Verschmelzen bei einer Temperatur von 140 bis 190°C vornimmt.

# INTERNATIONAL SEARCH REPORT

International Application No PCT/SU 90/00082

| I. CLASSIFICATION OF SUBJECT MATTER (if several classification symbols apply, indicate all) ⁶ |
|---|

According to International Patent Classification (IPC) or to both National Classification and IPC

Int.Cl.$^5$   C07C 53/126, 51/41//C08L21/00,7/00, C08K 5/09

| II. FIELDS SEARCHED |
|---|

**Minimum Documentation Searched ⁷**

| Classification System | Classification Symbols |
|---|---|
| Int.Cl.$^5$ | C07C 53/126,51/41,C08L21/00,7/00,C08 K 5/09 |

Documentation Searched other than Minimum Documentation
to the Extent that such Documents are Included in the Fields Searched ⁸

| III. DOCUMENTS CONSIDERED TO BE RELEVANT ⁹ | | |
|---|---|---|
| Category * | Citation of Document, ¹¹ with indication, where appropriate, of the relevant passages ¹² | Relevant to Claim No. ¹³ |
| A | US, A, 4340515 (AKZO NV ), 20 July 1982 (20.07.82), sample 1, claims 1-9 | 1-4 |
| A | EP, B1, 0148782 (MANCHEM LIMITED ASHTON NEW ROAD ), 23 September 1987 (23.09.87),samples 1-2, claims 1-3,5,9 | 1-4 |
| A | FR, A1, 2575745 (SOCIETE CHIMIQUE DES CHARBONNAGES), 11 July 1986 (11.07.86), sample 1,the claims | 2-3 |
| A | JP, A, 59-170035, 26 September 1984 (26.09.84) | 1-2 |

* Special categories of cited documents: ¹⁰
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art.
"&" document member of the same patent family

| IV. CERTIFICATION | |
|---|---|
| Date of the Actual Completion of the International Search | Date of Mailing of this International Search Report |
| 12 July 1990 (12.07.90) | 17 July 1990 (17.07.90) |
| International Searching Authority | Signature of Authorized Officer |
| ISA/SU | |

Form PCT/ISA/210 (second sheet) (January 1985)